# EUROPÄISCHE PATENTSCHRIFT

(11) **EP 3 035 915 B1**
(45) Veröffentlichungstag und Bekanntmachung des Hinweises auf die Patenterteilung: **22.08.2018**
(21) Anmeldenummer: 14776998.8
(22) Anmeldetag: 16.09.2014
(51) Int. Cl.: A61K 9/00, A61K 47/38, A61K 31/785, A61K 31/167, A61K 31/245, A61K 31/445, A61P 31/00, A61P 23/02, A61M 5/178, A61K 45/06, A61M 25/00

(54) **ZUSAMMENSETZUNG, INSBESONDERE IN FORM EINES GLEITGELS ENTHALTEND EIN LOKALANÄSTHETIKUM UND POLYHEXANID**
COMPOSITION, ESPECIALLY IN THE FORM OF A LUBRICANT GEL COMPRISING A LOCAL ANAESTHETIC AND POLYHEXANIDE
COMPOSITION, EN PARTICULIER SOUS LA FORME D'UN GEL LUBRIFIANT CONTENANT UN ANESTHÉSIQUE LOCAL ET DU POLYHEXANIDE

(30) Priorität: 21.11.2013 DE 102013112876; 13.01.2014 DE 102014100274
(43) Veröffentlichungstag der Anmeldung: 29.06.2016
(73) Patentinhaber: Farco-Pharma GmbH, 50670 Köln (DE)
(72) Erfinder: VESTWEBER, Anna-Maria, 51399 Burscheid (DE); MEIER, Andreas, 13629 Berlin (DE)
(74) Vertreter: Von Rohr Patentanwälte Partnerschaft mbB
(86) Internationale Anmeldenummer: PCT/EP2014/002492
(87) Internationale Veröffentlichungsnummer: WO 2015/074730

(56) Entgegenhaltungen:
- EP-A1- 2 415 487

## Beschreibung

Die vorliegende Erfindung betrifft das Gebiet der Medizin bzw. Medizintechnik. Insbesondere betrifft die vorliegende Erfindung das Gebiet der medizinisch einsetzbaren Katheter, welche insbesondere in Form von Harnblasenkathetern und/oder Fistelkathetern vorliegen können, sowie den Bereich der insbesondere zur Erleichterung bzw. Verbesserung der Katheterisierung verwendbaren Zusammensetzungen bzw. Gleitmittel. Die vorliegende Erfindung betrifft auch den Bereich der Endoskopie, insbesondere im Zusammenhang mit dem medizinischen Einsatz von Endoskopien oder medizinischen Sonden.

Die vorliegende Erfindung betrifft insbesondere eine Zusammensetzung, insbesondere pharmazeutische Zusammensetzung, vorzugsweise in Form eines Gleitgels und/oder Gleitmittels, welche als (Lokal-)Antiseptikum und/oder (Lokal-)-Anästhetikum, verwendet werden kann bzw. welches bei diagnostischen und/oder therapeutischen (operativen) Eingriffen, vorzugsweise bei Katheterisierungen, Sondierungen, Endoskopien, Intubationen und/oder geburtshilflichen Eingriffen oder dergleichen, verwendet werden kann.

Im Rahmen von medizinischen Eingriffen zu Zwecken der Diagnose, Therapie oder Prophylaxe ist es oftmals erforderlich, medizinische Geräte, wie Katheter, über vorzugsweise natürliche, aber auch über künstliche bzw. operativ hergestellte Körperöffnungen in den menschlichen oder tierischen Körper einzuführen. Zu derartigen Eingriffen zählen beispielsweise Katheterisierungen der Harnblase, wobei diese transurethral oder perkutan bzw. suprapubisch durchgeführt werden können, Sondierungen, Endoskopien, Intubationen sowie geburtshilfliche Eingriffe.

Im Bereich der Urologie werden häufig Katheter im Rahmen von Diagnose- und Therapieverfahren verwendet, beispielsweise um Urin aus der Harnblase bzw. der Niere abzuleiten oder um Medikamente bzw. Kontrastmittel in den Urogenitaltrakt einzubringen. Hierbei wird zwischen perkutanen, insbesondere suprapubischen Kathetern, welche durch die Haut in die Blase bzw. in das Nierenbecken geführt werden, und transurethralen Kathetern, welche entlang der Harnröhre bzw. durch die Harnröhre in den Körper bzw. in die Blase eingeführt werden, unterschieden. Perkutane bzw. suprapubische Blasen- und Nierenkatheter werden in erster Linie bei einer längeren Verweildauer des Katheters oder in Fällen, in welchen der Einsatz eines transurethralen Katheters zu risikobehaftet ist, verwendet. Transurethrale Katheter werden dagegen bevorzugt als Einmalkatheter bei diagnostischen Verfahren bzw. als Dauerkatheter im Allgemeinen mit einer Verweilzeit von wenigen Tagen verwendet.

Transurethrale Katheter besitzen den Vorteil, dass sie bei sachgerechter Verwendung wesentlich schonender für den Patienten sind, insbesondere da mit dem Legen des Katheters einhergehende Verletzungen minimiert werden. Dennoch ist das Legen eines transurethralen Katheters - die sogenannte Katheterisierung - ein oftmals als sehr unangenehm oder sogar als schmerzhaft beschriebener Vorgang, bei welchem darüber hinaus die Gefahr von Verletzungen des Urogenitaltraktes besteht. Zur Erleichterung der Katheterisierung werden im Stand der Technik Hilfsmittel, wie beispielsweise Gleitmittel, verwendet. Diese sind jedoch hinsichtlich der angestrebten Verbesserung der Katheterisierung sowie in Bezug auf die Gewährleistung geringerer Nebenwirkungen während des Verbleibs des Katheters im Körper oftmals nur unzulänglich.

Auch um eine Reibungsminderung zwischen medizinischen Geräten, wie Kathetern bzw. Sonden oder Endoskopen, und Haut bzw. Schleimhaut bzw. der Körperöffnung zu erreichen, hat es sich als vorteilhaft erwiesen, Gleitmittel bzw. Gleitgele als Hilfsmittel einzusetzen, um auf diese Weise Hautirritationen, Schmerzen und Verletzungen infolge des Eingriffs bzw. der Katheterisierung vorzubeugen. Darüber hinaus ist es im Zusammenhang mit Eingriffen der vorgenannten Art vorteilhaft, wenn gleichzeitig eine Desinfektion der Haut bzw. Schleimhaut im behandelten Areal erfolgt, um Infektionen und Entzündungen vorzubeugen.

Gleitmittel der ersten Generation basieren dabei vorwiegend auf fettigen bzw. öligen Substanzen, was jedoch in vielerlei Hinsicht nachteilig ist, insbesondere im Hinblick auf die Keimfreiheit und die Benetzungseigenschaften. Inzwischen bestehen Gleitmittel üblicherweise aus einem fettfreien Gel, welches zur Keimzahlreduktion gegebenenfalls einen antiseptischen Wirk- und/oder Inhaltsstoff enthalten kann. Darüber hinaus ist es möglich, dass Gleitmittel bzw. Gleitgele ein Lokalanästhetikum enthalten, um die Schmerzen während des Eingriffs zu lindern.

Die aus dem Stand der Technik bekannten Gleitmittel bzw. Gleitgele weisen zwar gegenüber den Gleitmitteln der ersten Generation eingesetzten öl- und fettbasierten Gleitmitteln mitunter verbesserte Benetzungs- bzw. Gleiteigenschaften auf, sind jedoch bezüglich ihrer Wirkung und Anwendung nicht immer optimal, insbesondere im Hinblick auf die Wirksamkeit bzw. den Wirkeintritt und die Verträglichkeit. Vor allem die Wirkung des gegebenenfalls eingesetzten Lokalanästhetikums ist nicht immer ausreichend, so dass keine zufriedenstellende bzw. ausreichende Schmerzlinderung erfolgt. Insbesondere liegt oftmals keine optimale Abstimmung mit den weiteren Komponenten des Gleitmittels vor, so dass die Wirkeffizienz des Lokalanästhetikums als solches insbesondere hinsichtlich seiner Wirksamkeit bzw. Verfügbarkeit am Wirkort nicht immer optimiert ist.

Auch hinsichtlich der antimikrobiellen bzw. antiseptischen Eigenschaften sind die Gleitmittel des Standes der Technik nicht immer zufriedenstellend, was insbesondere auch mit den üblicherweise eingesetzten antiseptischen Wirk- und/oder Inhaltsstoffen zusammenhängt. So werden im Stand der Technik als antiseptische Komponenten in Gleitgelen bzw. Gleitmitteln oftmals Chlorhexidin oder Octenidin eingesetzt. Nachteilig hierbei ist jedoch die nicht immer optimale Verträglichkeit. Zwar ist die Wirksamkeit insgesamt zumindest ausreichend, jedoch nicht immer optimal, da der Wirkeintritt mitunter verzögert erfolgt und darüber hinaus oftmals nicht langanhaltend genug ist.

Weiterhin ist insbesondere der Einsatz von Chlorhexidin unter bestimmten Umständen mit einer Ausbildung von Resistenzen gegenüber verschiedenen Mikroorganismen verbunden, was in Anbetracht der häufig auftretenden Krankenhaus- bzw. Hospitalkeime in mehrfacher Hinsicht nachteilig ist. Mitunter wird bereits durch den bloßen Einsatz von Chlorhexidin die Entstehung weiterer resistenter Mikroorganismen gefördert, und darüber hinaus besteht kein wirkungsvoller Schutz gegenüber bereits resistenten Erregern bzw. Mikroorganismen.

Insbesondere Chlorhexidin weist bei bestimmten Keimen der Spezies *Staphylococcus aureus* keine bzw. nahezu keine Wirkung auf, was auch durch eine Konzentrationssteigerung des Wirkstoffs nicht kompensiert werden kann. Zudem ist es auch kaum möglich, Biofilme mit Chlorhexidin bzw. Octenidin wirkungsvoll zu bekämpfen bzw. aufzubrechen. Darüber hinaus besitzen Chlorhexidin und Octenidin insbesondere bei hoher Konzentration bzw. Fehlanwendungen eine gewisse Zytotoxizität, was insbesondere im Hinblick auf die Wundheilung bzw. Wundantiseptik problematisch sein kann. Insbesondere die Wundheilung wird durch Octenidin und Chlorhexidin unter solchen Umständen verlangsamt.

Die EP 2 415 487 A1 betrifft eine Zusammensetzung zur Verwendung bei Ultraschalluntersuchungen, welche Stärke, ein schnell wirkendes Lokalanästhetikum bzw. dessen pharmazeutisch verträgliche Salze, ein Lokalanästhetikum mit langanhaltender Wirkung sowie einen antiseptischen Wirkstoff bzw. dessen Salze aufweist.

Weiterhin betrifft die Publikation gemäß Doherty: "Instillagel: an anaesthetic antiseptic gel for use in catheterization" eine Katheterisierungszusammensetzung, welche als anästhetisches Gel ausgebildet ist und als Wirkstoffe 2 Gew.-% Lidocain und 0,25 Gew.-% Chlorhexidingluconat-Lösung enthält.

Somit sind die im Stand der Technik bekannten Gleitmittel bzw. Gleitgele in Bezug auf ihre Verträglichkeit sowie ihre Wirkeffizienz, insbesondere was die Wundantiseptik bzw. die antiseptische Wirkung sowie die Schmerzlinderung anbelangt, nicht immer optimal.

Vor diesem Hintergrund liegt der vorliegenden Erfindung die Aufgabe zugrunde, verbesserte Zusammensetzungen zur Verwendung als Gleitmittel bzw. Gleitgele bereitzustellen, welche die zuvor geschilderten Nachteile der im Stand der Technik bekannten Gleitmittel bzw. Gleitgele zu verhindern bzw. zumindest zu verringern imstande sind.

Eine weitere der vorliegenden Erfindung zugrundeliegende Aufgabe ist darin zu sehen, verbesserte Zusammensetzungen in Form von Gleitmitteln bzw. Gleitgelen bereitzustellen, welche gegenüber den Zusammensetzungen des Standes der Technik verbesserte antiseptische Eigenschaften sowie keine oder zumindest verringerte Nebenwirkungen und einen verbesserten bzw. beschleunigten Wirkeintritt aufweisen. Darüber hinaus sollen gemäß der vorliegenden Erfindung Zusammensetzungen bereitgestellt werden, welche verbesserte schmerzlindernde Eigenschaften besitzen.

Zur Lösung der zuvor geschilderten Aufgabe schlägt die vorliegende Erfindung eine Zusammensetzung gemäß Anspruch 1 vor; weitere vorteilhafte Ausgestaltungen sind Gegenstand der diesbezüglich abhängigen Ansprüche. Der Geltungsbereich der Erfindung wird von den beigefügten Ansprüchen definiert. Weitere Offenbarung in der vorliegenden Anmeldung stellt keinen Teil der vorliegenden Erfindung dar.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.
Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.
Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.
Gegenstand der vorliegenden Erfindung ist somit - gemäß einem **ersten** Aspekt der vorliegenden Erfindung - eine Zusammensetzung zur Verwendung bei der prophylaktischen und/oder therapeutischen und/oder diagnostischen Schleimhautdesinfektion im Rahmen von transurethralen oder suprapubischen Katheterisierungen, Sondierungen, Endoskopien, Intubationen und/oder geburtshilflichen Eingriffen, wobei die Zusammensetzung in Form eines Gleitgels und/oder Gleitmittels vorliegt,
wobei die Zusammensetzung jeweils in wirksamen, insbesondere pharmazeutisch wirksamen Mengen
(a) mindestens ein Lokalanästhetikum als "Komponente (a)" und
(b) Polyhexanid und/oder dessen Salze als "Komponente (b)", insbesondere als antiseptischen Wirk- und/oder Inhaltsstoff (Desinfektionsmittel),
enthält,
wobei die Zusammensetzung frei ist von Polyalkylenglykolen und Alkylenglykolen undwobei die Zusammensetzung einen physiologisch verträglichen pH-Wert im Bereich von 5 bis 7,5 aufweist.

Denn die Anmelderin hat im Rahmen der vorliegenden Erfindung in überraschender Weise gefunden, dass die gezielte Kombination von Polyhexanid als antiseptischem Wirk- und/oder Inhaltsstoff (Desinfektionsmittel) zusammen mit mindestens einem Lokalanästhetikum zu Zusammensetzungen, vorzugsweise zur Verwendung als Gleitmittel bzw. -gel bei Katheterisierungen oder dergleichen, führt, welche gegenüber Gleitmitteln bzw. Gleitgelen des Standes der Technik signifikant verbesserte Eigenschaften aufweisen, insbesondere im Hinblick auf die Verträglichkeit sowie einen schnellen Wirkeintritt bei verlängerter Wirkdauer und gleichzeitig hervorragenden schmerzlindernden bzw. analgetischen Eigenschaften.

Die erfindungsgemäßen Zusammensetzungen als solche eignen sich zudem insbesondere zur prophylaktischen bzw. therapeutischen (Schleim-)Hautdesinfektion, insbesondere im Zusammenhang mit vorzugsweise in der Diagnose und/oder Therapie durchgeführten Katheterisierungen, Endoskopien oder dergleichen.

Die Begriffe "pharmazeutische Zusammensetzung", "pharmazeutische Zubereitung" oder dergleichen, wie sie im Rahmen der vorliegenden Erfindung verwendet werden, sind sehr umfassend zu verstehen und bezeichnen nicht nur pharmazeutische Präparate bzw. Pharmazeutika oder Arzneimittel als solche bzw. im engeren Sinne, sondern auch sogenannte Medizinprodukte, homöopathische Mittel, Lebensmittel, Nahrungsergänzungsmittel oder dergleichen.

Zudem bezieht sich der erfindungsgemäß verwendete Begriff "(Schleim-)Hautdesinfektion" insbesondere auf eine bei der vorzugsweise therapeutischen bzw. diagnostischen Anwendung von Kathetern, Endoskopen oder dergleichen vorliegende bzw. durchgeführte Desinfektion bzw. Keimreduktion insbesondere von in Kontakt mit den eingesetzten Kathetern bzw. Endoskopen stehenden Körperbereichen, wie Haut bzw. Schleimhaut, und zwar insbesondere zur Vermeidung bzw. Verminderung der Ausbildung von Biofilmen, zur Verhinderung von Infektionen bzw. zur Verhinderung der Übertragung von insbesondere pathogenen Keimen bzw. Erregern von einem eingesetzten medizinischen Gerät, wie einem Katheter, auf die damit in Kontakt stehende Haut bzw. Schleimhaut.

Wie nachfolgend erläutert, weist die erfindungsgemäße Zusammensetzung weitere zahlreiche Vorteile und Besonderheiten auf:
Die erfindungsgemäßen Zusammensetzungen kommen - insbesondere durch die deutlich verbesserten antiseptischen bzw. antimikrobiellen Eigenschaften, welche maßgeblich durch den Einsatz von Polyhexanid hervorgerufen werden - ohne den zusätzlichen Einsatz von Konservierungsstoffen aus. Dadurch wird die Verträglichkeit der erfindungsgemäßen Zusammensetzungen weitergehend erhöht, so dass unerwünschte systemische Reaktionen bzw. Reizungen sowie allergische Reaktionen auf etwaige Konservierungsmittel ausgeschlossen bzw. minimiert werden können.

Gemäß einer erfindungsgemäßen Ausführungsform ist die Zusammensetzung nach der Erfindung ohne Konservierungsmittel bzw. -stoffe ausgebildet bzw. ist zumindest im Wesentlichen frei von Konservierungsmittel bzw. -stoffen.

Auf Basis der von der Anmelderin durchgeführten Anwendungs- und Wirksamkeitsstudien wird darüber hinaus deutlich, dass sich das Antiseptikum bzw. Desinfektionsmittel Polyhexanid einerseits sowie die erfindungsgemäß eingesetzten Lokalanästhetika, insbesondere Lidocain, andererseits in Bezug auf ihre Wirksamkeit in synergistischer Weise ergänzen bzw. verstärken. Zum einen wird eine hervorragende antimikrobielle Wirkleistung, insbesondere ein schneller Wirkeintritt und eine lang anhaltende Wirkdauer, erzielt, zum anderen lassen sich durch den Einsatz der erfindungsgemäßen Zusammensetzungen auch Schmerzen bei Eingriffen, wie Katheterisierungen, Sondierungen, Endoskopien, Intubationen bzw. geburtshilflichen Eingriffen, signifikant lindern bzw. verringern.

Darüber hinaus ist auch der Einsatz als solcher von Polyhexanid als Antiseptikum bzw. als antiseptischer Wirk- und/oder Inhaltsstoff (Desinfektionsmittel) in mehrfacher Hinsicht vorteilhaft. Insbesondere lässt sich die Wirkeffizienz signifikant verbessern, da Polyhexanid einen besonders schnellen Wirkeintritt ermöglicht, die Wirkung darüber hinaus aber auch äußerst lange anhält. Weiterhin erfolgt bei dem Einsatz von Polyhexanid als Schleimhautdesinfektionsmittel keine relevante systemische Resorption des Wirkstoffs, so dass das Risiko für ein Auftreten von Nebenwirkungen deutlich gesenkt werden kann. In diesem Zusammenhang ist auch hervorzuheben, dass Polyhexanid nicht zytotoxisch wirkt, was die Verträglichkeit der gesamten Zusammensetzung weitergehend steigert.

Zudem besitzt das erfindungsgemäß eingesetzte Antiseptikum bzw. der antiseptische Wirk- und/oder Inhaltsstoff (Desinfektionsmittel) in Form von Polyhexanid ein besonders breites Wirkspektrum, d.h. es wirkt sowohl gegen Gram-positive als auch gegen Gram-negative Erreger. Dies ist insbesondere dahingehend von Vorteil, dass Polyhexanid auch eine gute Wirkeffizienz gegenüber den sogenannten Krankenhaus- bzw. Hospitalkeimen, wie *Staphylococcus aureus,* besitzt. Ohne sich hierbei auf diese Theorie beschränken zu wollen, wird aufgrund der unspezifischen Wirkweise bzw. Interaktion von Polyhexanid mit den sauren Membranphospholipiden der Bakterien bzw. Mikroorganismen verhindert, dass gegenüber Polyhexanid Resistenzen ausgebildet werden können.

Zudem ist die Lagerstabilität der erfindungsgemäßen Zusammensetzungen durch den zweckgerichteten Einsatz von Polyhexanid gesteigert, da dieser antiseptische Wirk- und/oder Inhaltsstoff (Desinfektionsmittel) als solcher bereits eine hervorragende mikrobielle Stabilität besitzt, so dass ein Einsatz von (zusätzlichen) Konservierungsmitteln nicht erforderlich ist.

Weiterhin ist es vorteilhaft, dass durch den Einsatz von Polyhexanid die Wundheilung nicht negativ beeinflusst wird, vielmehr wird die Wundheilung durch die hohe antimikrobielle Aktivität von Polyhexanid sogar gefördert.

Darüber hinaus hat sich im Rahmen der vorliegenden Erfindung überraschend gezeigt, dass die erfindungsgemäßen Zusammensetzungen in Bezug auf ihre Verträglichkeit verbessert werden können, wenn auf den Einsatz von Glykolen, insbesondere Propylenglykol, verzichtet wird, da diese bzw. dieses zu Unverträglichkeiten, wie Schleimhautirritationen und allergischen Reaktionen, führen können bzw. kann. Somit kann die erfindungsgemäße Zusammensetzung gemäß einer erfindungsgemäßen Ausführungsform zumindest im Wesentlichen frei von Glykolen, insbesondere frei von Propylenglykolen, sein.

Zudem zeichnet sich die erfindungsgemäße Zusammensetzung durch eine speziell abgestimmte bzw. eingestellte Viskosität und hervorragende Schmier- bzw. Gleiteigenschaften aus, so dass die erfindungsgemäße Zusammensetzung in hervorragender Weise an der Schleimhaut haftet und diese benetzt, was ein problemloses Gleiten bzw. Einführen von medizinischen Geräten, wie Kathetern, Sonden, Endoskopen oder Ultraschallköpfen, ermöglicht. Somit wird der in Kontakt mit dem medizinischen Gerät stehende Körperbereich, wie Haut bzw. Schleimhaut, weitestgehend vor Verletzungen geschützt. Durch das zusätzliche Lokalanästhetikum wird - im Zusammenwirken mit bzw. zusätzlich zu den außerordentlich guten Haft- und Schmiereigenschaften der erfindungsgemäßen Zusammensetzung - zudem erreicht, dass die zugrundeliegenden Eingriffe für die Patienten äußerst schonend und schmerzfrei verlaufen.

Die erfindungsgemäßen Zusammensetzungen in Form von Gleitmitteln bzw. Gleitgelen sind derart konzipiert, dass sie die Schleimhäute optimal benetzen und an diesen haften und zudem hervorragende Schmierstoffeigenschaften aufweisen. Auf diese Weise sind diagnostische bzw. therapeutische Eingriffe sehr viel einfacher und schneller durchzuführen, und das Risiko von Verletzungen der Schleimhaut im Bereich des untersuchten Gewebes bzw. Körperteils wird gleichzeitig minimiert, wie zuvor angeführt.

Bei den nachfolgend aufgeführten Substanzen, welche in der erfindungsgemäßen Zusammensetzung enthalten sein können, sind stets die Verbindungen genannt, welche der Zusammensetzung beigemischt bzw. zugegeben werden können. Hierbei ist es möglich, dass die Verbindungen durch Reaktionen in andere Substanzen überführt werden, insbesondere sind Säure/Base-Reaktionen möglich, d.h. die eingesetzten Substanzen können beispielsweise protoniert oder deprotoniert werden. Dies ist dem Fachmann als solches jedoch bekannt.

Das erfindungsgemäß eingesetzte Polyhexanid besitzt eine antiseptische bzw. antimikrobielle Wirkung. In chemischer Hinsicht handelt es sich im Allgemeinen bei dem Wirkstoff Polyhexanid um Polyhexamethylenbiguanid (PHMB), welcher synonym auch als Poly(iminocarbonylimidoyl-iminocarbonylimidoylimino-1,6-hexandiyl)-hydrochlorid oder Polyhexanid bezeichnet werden kann und insbesondere die Summenformel C₈H₁₇N₅ sowie ein Molekulargewicht von 183,25 g/mol aufweist.

Vorteilhaft in Bezug auf Polyhexanid ist insbesondere sein breites Wirkungsspektrum, welches auch gegen die schwer zu behandelnden Krankenhauskeime, wie beispielsweise *Staphylococcus aureus,* gerichtet ist. Darüber hinaus wirkt Polyhexanid bereits in äußerst geringen Konzentrationen, da es direkt an die bakterielle Zellwand bindet und diese derart schädigt, dass es üblicherweise zum Zelltod kommt. Auf diese Weise wird der wirksame Einsatz dieses Antiseptikums auch in geringen Konzentrationen ermöglicht, was das Risiko für das Auftreten von Nebenwirkungen entsprechend senkt. Darüber hinaus zeichnet sich Polyhexanid durch eine geringe systemische Resorption aus, was gleichermaßen zu einer Minimierung von Nebenwirkungen führt.

Unter dem Begriff "Lokalanästhetika", wie dieser erfindungsgemäß verwendet wird, werden im Rahmen der vorliegenden Erfindung im Allgemeinen Anästhetika mit örtlich begrenzter Wirkung bzw. zur örtlichen Schmerzstillung bzw. Betäubung verstanden, insbesondere wobei die im Rahmen der erfindungsgemäßen Verwendung eingesetzten Lokalanästhetika keine euphorisierende oder suchterzeugende Wirkung aufweisen sollten. Die chemische Struktur der zugrundeliegenden Lokalanästhetika ist grundsätzlich ähnlich. Sie umfasst im Allgemeinen eine lipophile aromatische Ringstruktur, eine Zwischenkette und eine hydrophile Aminogruppe. Nach der Zwischenkette unterscheidet man Aminoester ("Estertyp") und Aminoamide ("Amidtyp"). Die Aminoester werden im Gewebe durch eine Cholinesterase metabolisiert, der Abbau der Aminoamide erfolgt in der Leber durch N-Dealkylierung oder Hydrolyse. Insbesondere entfalten - ohne sich auf diese Theorie beschränken zu wollen - Lokalanästhetika ihre Wirkung an der Zellmembran von Nervenzellen, wobei die Bildung und Fortleitung von Empfindungen, wie Temperatur, Druck oder Schmerz, lokal abgeschwächt bzw. vollständig unterbrochen wird.

In Bezug auf das erfindungsgemäß eingesetzte Lokalanästhetikum können im Rahmen der vorliegenden Erfindung eine Reihe lokalanästhetisch wirksamer Substanzen, welche dem Fachmann an sich wohlbekannt sind, verwendet werden. Erfindungsgemäß ist es jedoch bevorzugt, wenn das Lokalanästhetikum aus der Gruppe von Lokalanästhetika vom Estertyp, insbesondere Aminoestern, und Lokalanästhetika vom Amidtyp, insbesondere Aminoamiden, sowie deren Mischungen oder Kombination ausgewählt wird, insbesondere aus Lidocain, Mepivacain, Prilocain, Bupivacain, Articain und Ropivacain sowie deren Mischungen oder Kombination, besonders bevorzugt Lidocain.

Im Rahmen der vorliegenden Erfindung werden besonders gute Ergebnisse erzielt, wenn das Lokalanästhetikum Lidocain ist. Hierbei handelt es sich um ein Lokalanästhetikum vom Amidtyp, welches über eine gute Wirksamkeit bei gleichzeitig schnellem Wirkeintritt verfügt. Für weitere Ausführungen in Bezug auf Lidocain kann beispielsweise auf Römpp Chemielexikon, 10. Auflage, Band 3, 1997, Georg Thieme Verlag Stuttgart/New York, Stichwort: "Lidocain" sowie auf die dort jeweils in Bezug genommene Literatur verwiesen werden, deren gesamter Inhalt hiermit durch Bezugnahme eingeschlossen ist.

Was die eingesetzte Menge des Lokalanästhetikums bzw. der Komponente (a) anbelangt, so kann diese in weiten Bereichen variieren. Eine besonders gute Wirksamkeit hat sich im Rahmen der vorliegenden Erfindung jedoch gezeigt, wenn die Zusammensetzung die Komponente (a) in einer Menge im Bereich von 0,1 bis 10 Gew.-%, insbesondere im Bereich von 0,5 bis 8 Gew.-%, vorzugsweise im Bereich 0,7 bis 6 Gew.-%, bevorzugt im Bereich von 1 bis 3 Gew.-%, bezogen auf die Zusammensetzung, enthält.

Gleichermaßen kann es vorgesehen sein, dass die Zusammensetzung die Komponente (a) in einer Menge von mindestens 0,01 Gew.-%, insbesondere mindestens 0,1 Gew.-%, vorzugsweise mindestens 0,5 Gew.-%, bevorzugt mindestens 1 Gew.-%, bezogen auf die Zusammensetzung, enthält. In diesem Zusammenhang kann es auch vorgesehen sein, dass die Zusammensetzung die Komponente (a) in einer Menge von höchstens 15 Gew.-%, insbesondere höchstens 10 Gew.-%, vorzugsweise höchstens 8 Gew.-%, bevorzugt höchstens 5 Gew.-%, besonders bevorzugt höchstens 3 Gew.-%, bezogen auf die Zusammensetzung, enthält.

Auch die Menge des erfindungsgemäß eingesetzten Polyhexanids als Antiseptikum kann in weiten Bereichen variieren. In Bezug auf die antiseptische bzw. antimikrobielle Wirkung werden erfindungsgemäß die besten Ergebnisse erzielt, wenn die Zusammensetzung Polyhexanid und/oder dessen Salze in einer Menge im Bereich von 0,05 bis 10 Gew.-%, insbesondere im Bereich von 0,1 bis 8 Gew.-%, vorzugsweise im Bereich von 0,5 bis 5 Gew.-%, bevorzugt im Bereich von 0,75 bis 3 Gew.-%, besonders bevorzugt im Bereich von 1 bis 2 Gew.-%, bezogen auf die Zusammensetzung, enthält.

Auch kann es im Rahmen der vorliegenden Erfindung vorgesehen sein, dass die Zusammensetzung Polyhexanid und/oder dessen Salze in einer Menge von mindestens 0,05 Gew.-%, insbesondere mindestens 0,1 Gew.-%, vorzugsweise mindestens 0,5 Gew.-%, bevorzugt mindestens 0,75 Gew.-%, besonders bevorzugt mindestens 1 Gew.-%, bezogen auf die Zusammensetzung, enthält. Gleichermaßen kann es in Bezug auf die eingesetzte Menge von Polyhexanid vorgesehen sein, dass die Zusammensetzung das Polyhexanid in einer Menge von höchstens 10 Gew.-%, insbesondere höchstens 8 Gew.-%, vorzugsweise höchstens 5 Gew.-%, bevorzugt höchstens 3 Gew.-%, besonders bevorzugt höchstens 2 Gew.-%, bezogen auf die Zusammensetzung, enthält.

Darüber hinaus hat es sich im Rahmen der vorliegenden Erfindung in überraschender Weise gezeigt, dass in Bezug auf eine Wirkoptimierung nicht nur die Wirkstoffmenge der beiden eingesetzten Wirkstoffe als solche von Bedeutung ist, sondern auch das eingesetzte Mengenverhältnis beider Wirkstoffe zueinander. Eine besondere gute Wirkeffizienz im Hinblick auf die antiseptische Wirkung einerseits sowie die schmerzlindernde Wirkung andererseits wird im Rahmen der vorliegenden Erfindung erzielt, wenn die Zusammensetzung die Komponente (a) und die Komponente (b) in einem gewichtsbezogenen Verhältnis von [(a) : (b)] im Bereich von 1 : 50 bis 50 : 1, insbesondere im Bereich von 1 : 20 bis 20 : 1, vorzugsweise im Bereich von 1 : 10 bis 10 : 1, bevorzugt im Bereich von 1 : 5 bis 5 : 1, besonders bevorzugt 1 : 3 bis 3 : 1, enthält. Denn durch den Einsatz beider Wirkstoffe in definiertem Mengenverhältnis zueinander geht die Wirkung von Lokalanästhetikum einerseits und Antiseptikum andererseits bei kombiniertem Einsatz über die jeweilige Einzelwirkung der Wirkstoffe bei alleiniger Verabreichung hinaus, was als Indiz für das Vorliegen eines synergistischen Effekts zu werten ist.

Die erfindungsgemäße Zusammensetzung kann insgesamt variabel ausgestaltet werden. Bevorzugte Ausführungsformen sind nachfolgend im Detail erläutert.

Gemäß einer erfindungsgemäß bevorzugten Ausführungsform kann es vorgesehen sein, dass die Zusammensetzung wässrig oder wässrig-alkoholisch, vorzugsweise wässrig, basiert ist.

In diesem Zusammenhang ist es vorteilhaft, wenn die Zusammensetzung Wasser, insbesondere gereinigtes Wasser, in einer Menge im Bereich von 30 bis 99 Gew.-%, insbesondere im Bereich von 40 bis 98 Gew.-%, bevorzugt im Bereich von 50 bis 97 Gew.-%, besonders bevorzugt im Bereich von 60 bis 96 Gew.-%, bezogen auf die Zusammensetzung, enthält.

Darüber hinaus kann es im Rahmen der vorliegenden Erfindung vorgesehen sein, dass die Zusammensetzung nach der Erfindung mindestens einen vorzugsweise mehrwertigen Alkohol, insbesondere ausgewählt aus der Gruppe von Polyvinylalkoholen, Glycerin, Glykolen sowie deren Kombinationen, vorzugsweise Glycerin, enthält. Im Rahmen der vorliegenden Erfindung ist es vorgesehen, dass die Zusammensetzung frei ist von (Poly-)Alkylenglykolen. Insbesondere ist es in diesem Zusammenhang vorgesehen, dass die erfindungsgemäße Zusammensetzung kein Propylenglykol aufweist. Denn (Poly-)Alkylenglykole, insbesondere Propylenglykol, können zu Hautirritationen und Unverträglichkeitsreaktionen führen, was insbesondere im Hinblick auf die Anwendung auf der Schleimhaut nachteilig ist, da die Schleimhaut besonders empfindlich Ist. Darüber hinaus wird durch den Verzicht auf (Poly)-Alkylenglykole, insbesondere Propylenglykol, eine systemische, insbesondere eine übermäßige systemische Resorption der eingesetzten Wirkstoffe, d.h. des Lokalanästhetikums und des Antiseptikums, zumindest im Wesentlichen vermieden, was die Verträglichkeit der Zusammensetzung nach der Erfindung weitergehend steigert.

Was die eingesetzten Mengen des vorzugsweise mehrwertigen Alkohols anbelangt, so können diese in weiten Bereichen variieren. Besonders gute Ergebnisse werden erfindungsgemäß erhalten, wenn die Zusammensetzung den vorzugsweise mehrwertigen Alkohol in einer Menge im Bereich von 1 bis 80 Gew.-%, insbesondere im Bereich von 5 bis 70 Gew.-%, vorzugsweise im Bereich von 10 bis 60 Gew.-%, bevorzugt im Bereich von 15 bis 50 Gew.-%, bezogen auf die Zusammensetzung, enthält.

Gemäß einer weiteren erfindungsgemäßen Ausführungsform kann es vorgesehen sein, dass die Zusammensetzung eine Mischung oder ein Gemisch von Wasser und mindestens einem vorzugsweise mehrwertigen Alkohol aufweist. In diesem Zusammenhang hat es sich als vorteilhaft erwiesen, wenn der Anteil an Wasser mindestens 10 Gew.-%, insbesondere mindestens 30 Gew.-%, bevorzugt mindestens 50 Gew.-%, bezogen auf die Mischung oder das Gemisch, beträgt. Gleichermaßen kann es in Bezug auf diese erfindungsgemäße Ausführungsform vorgesehen sein, dass das gewichtsbezogene Verhältnis von Wasser zu Alkohol in der Zusammensetzung im Bereich von 9 : 1 bis 1 : 5, insbesondere im Bereich von 3 : 1 bis 1 : 3, vorzugsweise im Bereich von 2 : 1 bis 1 : 2, bevorzugt im Bereich von 1,5 : 1 bis 1 : 1,5, liegt.

Was darüber hinaus die Funktion der Zusammensetzung nach der Erfindung als Schmier- bzw. Gleitmittel anbelangt, ist es erfindungsgemäß vorgesehen, dass die Zusammensetzung viskos bzw. gelförmig ausgebildet ist bzw. in Form eines Gleitgels bzw. Gleitmittels vorliegt. Durch die viskose bzw. gelförmige Konsistenz wird die Eignung der erfindungsgemäßen Zusammensetzungen zur Reibungsminderung gewährleistet, d.h. der Widerstand zwischen Haut bzw. Schleimhaut einerseits sowie medizinischen Geräten andererseits wird verringert, so dass ein leichtes Einführen möglich ist, ohne dass Verletzungen oder Hautirritationen auftreten.

In diesem Zusammenhang ist es vorteilhaft, wenn die Zusammensetzung mindestens einen Gelbildner aufweist. Im Rahmen der vorliegenden Erfindung kann der Gelbildner aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen, vorzugsweise Cellulose und Cellulosederivaten, bevorzugt modifizierten Cellulosen, besonders bevorzugt chemisch modifizierten Cellulosen, ganz besonders bevorzugt Methylcellulose, Carboxymethylcellulose und/oder Hydroxyethylcellulose, noch mehr bevorzugt Hydroxyethylcellulose, ausgewählt sein.

Was die eingesetzte Menge des Gelbildners anbelangt, so kann diese in weiten Bereichen variiert werden. Insbesondere wird die Menge an Gelbildner in Abhängigkeit davon ausgewählt, welche Viskosität die erfindungsgemäße Zusammensetzung aufweisen soll. Im Rahmen der vorliegenden Erfindung hat es sich als vorteilhaft erwiesen, wenn die Zusammensetzung den Gelbildner in einer Menge im Bereich von 0,01 bis 20 Gew.-%, insbesondere im Bereich von 0,1 bis 10 Gew.-%, vorzugsweise im Bereich von 0,5 bis 5 Gew.-%, bevorzugt im Bereich von 1 bis 3 Gew.-%, bezogen auf die Zusammensetzung, aufweist.

Besonders leistungsfähige gelartige Strukturen bzw. Eigenschaften werden erhalten, wenn die erfindungsgemäße Zusammensetzung bei einer Temperatur von 20 °C eine dynamische Viskosität im Bereich von 20 bis 20.000 mPa·s, insbesondere im Bereich von 50 bis 15.000 mPa·s, vorzugsweise im Bereich von 100 bis 10.000 mPa·s, bevorzugt im Bereich von 500 bis 8.000 mPa·s, besonders bevorzugt im Bereich von 1.000 bis 7.000 mPa·s, noch mehr bevorzugt im Bereich von 2.000 bis 6.000 mPa·s, aufweist.

Im Rahmen der vorliegenden Erfindung ist es somit möglich, auf Basis der ausgewählten Gelbildner sowie der einsetzbaren Mengen des Gelbildners die rheologischen Eigenschaften der Zusammensetzung nach der Erfindung gezielt einzustellen und sozusagen maßschneidern zu können.

Was die Methoden bzw. Verfahren zur Bestimmung der Viskosität der erfindungsgemäßen Zusammensetzung anbelangt, so sind diese als solche dem Fachmann bekannt, so dass keine weiteren diesbezüglichen Informationen an dieser Stelle notwendig sind. Insbesondere können Messgeräte wie Kapillarviskosimeter, Rotationsviskosimeter, Kugelfall-Viskosimeter oder Brookfield-Viskosimeter zur Bestimmung der Viskosität eingesetzt werden. Insbesondere beziehen sich die zuvor angegebenen Viskositätswerte auf eine Bestimmung gemäß DIN 53015 bei Raumtemperatur (20 °C) (z. B. mittels Kugeifall-Viskosimeter, z. B. RheoTec Messtechnik GmbH, Deutschland).

Darüber hinaus weist die erfindungsgemäße Zusammensetzung üblicherweise einen physiologisch verträglichen pH-Wert auf. Dabei ist es erfindungsgemäß vorgesehen, dass die Zusammensetzung einen pH-Wert im Bereich von 6 bis 7,5, bevorzugt im Bereich von 6 bis 7, aufweist.

Weiterhin ist es im Rahmen der vorliegenden Erfindung bevorzugt, wenn die Zusammensetzung eine physiologisch verträgliche elektrische Leitfähigkeit, insbesondere im Bereich von 1 bis 20 mS/cm, vorzugsweise im Bereich von 2 bis 15 mS/cm, bevorzugt im Bereich von 3 bis 13 mS/cm, besonders bevorzugt im Bereich von 5 zu 10 mS/cm, aufweist.

Darüber hinaus kann es im Rahmen der vorliegenden Erfindung vorgesehen sein, dass die Zusammensetzung außerdem mindestens eine Säure und/oder Base, insbesondere zur Einstellung des pH-Werts, vorzugsweise zur Einstellung eines physiologisch verträglichen pH-Werts, bevorzugt zur Ausbildung eines Puffersystems, enthält. Die diesbezüglichen Puffersysteme sind dem Fachmann wohlbekannt, so dass es hierzu keiner weiteren Ausführungen bedarf.

Es hat sich zudem als vorteilhaft erwiesen, wenn die Zusammensetzung vorzugsweise mindestens eine Base aufweist. Dabei kann es vorgesehen sein, dass die Base ausgewählt ist aus der Gruppe von Aminen, Carboxylaten, Alkali- und/oder Erdalkalimetallhydroxiden sowie deren Mischungen und Kombinationen, insbesondere Alkali- und Erdalkalimetallhydroxyden, bevorzugt Natriumhydroxid.

Der Zusatz von Säuren bzw. Basen, insbesondere von Basen, ermöglicht die gezielte Einstellung des pH-Werts, so dass die erfindungsgemäßen Zusammensetzungen physiologisch gut verträglich sind und gleichzeitig die bevorzugt eingesetzten Lokalanästhetika vorzugsweise vom Amidtyp protoniert vorliegen, wodurch die lokale Wirkung des Lokalanästhetikums verbessert bzw. gewährleistet wird. Gleichfalls kommt es zur Ausbildung von Puffersystemen, wodurch der pH-Wert der erfindungsgemäßen Zusammensetzung auch während der Applikation stabil bleibt.

Darüber hinaus ist es gemäß einer bevorzugten Ausführungsform der vorliegenden Erfindung vorgesehen, dass die erfindungsgemäße Zusammensetzung zumindest im Wesentlichen kein Konservierungsmittel bzw. keinen Konservierungsstoff aufweist. Insbesondere kann es im Rahmen der vorliegenden Erfindung vorgesehen sein, dass die Zusammensetzung zumindest im Wesentlichen frei ist von Konservierungsmitteln bzw. frei ist von Konservierungsstoffen. Durch den Verzicht auf Konservierungsmittel kann die Verträglichkeit der Zusammensetzungen noch weitergehend gesteigert werden. Hierbei ist es vollkommen überraschend, dass die erfindungsgemäße Zusammensetzung auch ohne den Einsatz von Konservierungsmitteln eine hervorragende Langzeitstabilität aufweist, und auch nach einer Lagerzeit von mindestens zwei Jahren kein mikrobiologischer Befall der Zusammensetzungen auftritt.

Weiterhin kann die erfindungsgemäße Zusammensetzung mindestens einen weiteren Wirk- und/oder Inhaltsstoff, insbesondere ausgewählt aus der Gruppe von Hautschutzmitteln, Antiseptika, Lokalanästhetika, Vitaminen, Spurenelementen, Mineralien, Mikronährstoffen sowie deren Kombinationen, enthalten.

Gleichermaßen kann es vorgesehen sein, dass die erfindungsgemäße Zusammensetzung mindestens einen üblichen pharmazeutischen Zusatz- und/oder Hilfsstoff enthält, welcher vorzugsweise aus der Gruppe von Verarbeitungshilfsstoffen, Stabilisatoren, Emulgatoren, Antioxidantien, Konservierungsstoffen, Feuchthaltemitteln, pH-Stellmitteln, pH-Puffersubstanzen, Verdickungsmitteln, Antiseptika, Färbe-, Puffer-, Riech-, Duft-, Streck-, Binde-, Netz- und/oder Konservierungsstoffen sowie deren Kombinationen ausgewählt ist.

Im Allgemeinen eignet sich die erfindungsgemäße Zusammensetzung zur Verwendung im Bereich der Medizin, Medizintechnik, Pharmazie und Kosmetik.

Insbesondere eignet sich die Zusammensetzung nach der Erfindung zur Verwendung als (Lokal-)Antiseptikum und/oder (Lokal-)Anästhetikum, insbesondere bei diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen.

Gleichermaßen eignet sich die erfindungsgemäße Zusammensetzung zur Verwendung bei der prophylaktischen bzw. therapeutischen (Schleimhaut-)Desinfektion, insbesondere im Rahmen von Katheterisierungen, vorzugsweise transurethralen oder suprapubischen Katheterisierungen, Endoskopien, Sondierungen, Intubationen sowie geburtshilflichen Eingriffen.

Zusammenfassend ist hervorzuheben, dass es erfindungsgemäß im Hinblick auf die Zusammensetzung nach der Erfindung erstmals gelungen ist, Gleitmittel bzw. Gleitgele als Hilfsmittel für therapeutische bzw. diagnostische bzw. prophylaktische Eingriffe, wie Katheterisierungen, Endoskopien, Sondierungen, Intubationen oder geburtshilfliche Eingriffe, bereitzustellen, welche den Gleitmitteln bzw. Gleitgelen des Standes der Technik deutlich überlegen sind. Die erfindungsgemäße Zusammensetzung weist insgesamt eine verbesserte Wirksamkeit, insbesondere eine gesteigerte antiseptische Wirkung, auf, und zwar sowohl im Hinblick auf das Wirkspektrum als auch im Hinblick auf einen schnellen Wirkeintritt. Darüber hinaus ist die Zusammensetzung in Bezug auf ihre Verträglichkeit verbessert, da insbesondere das eingesetzte Antiseptikum zumindest im Wesentlichen keine zytotoxische Wirkung besitzt und somit das Risiko unerwünschter Nebenreaktionen bzw. -wirkungen minimiert wird. Darüber hinaus ergänzen sich das eingesetzte Lokalanästhetikum einerseits sowie das Antiseptikum andererseits in synergistischer Weise zueinander, d.h. der kombinierte Einsatz beider Wirkstoffe, insbesondere in definierten Mengenverhältnissen zueinander, führt zu einer signifikanten Wirkungssteigerung in Bezug auf beide Wirkstoffe. Darüber hinaus weist die erfindungsgemäße Zusammensetzung maßgeschneiderte bzw. im Hinblick auf den jeweiligen Einsatzbereich optimierte Viskositäts- und Haftungseigenschaften auf, so dass medizinische Eingriffe, insbesondere Katheterisierungen, Endoskopien, Intubationen, Sondierungen sowie geburtshilfliche Eingriffe, leichter und schonender - insbesondere mit verringertem Verletzungsrisiko - durchgeführt werden können.

Die erfindungsgemäße Zusammensetzung eignet sich somit für die Verwendung als (Lokal-)Antiseptikum und/oder (Lokal-)Anästhetikum, insbesondere bei diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen.

Somit betrifft die vorliegende Erfindung die erfindungsgemäße Zusammensetzung als solche, wie zuvor beschrieben, zur Verwendung als (Lokal-)Antiseptikum und/oder als (Lokal-)Anästhetikum, insbesondere bei diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen.

Gleichermaßen betrifft die vorliegende Erfindung auch die Verwendung der erfindungsgemäßen Zusammensetzung zur prophylaktischen und/oder therapeutischen (Schleim-)Hautdesinfektion, insbesondere im Rahmen von Katheterisierungen, vorzugsweise transurethralen oder suprapubischen Katheterisierungen, Sondierungen, Endoskopien, Intubationen und/oder geburtshilflichen Eingriffen.

Somit betrifft die vorliegende Erfindung auch die erfindungsgemäße Zusammensetzung als solche, wie zuvor beschrieben, zur Verwendung bei der prophylaktischen und/oder therapeutischen (Schleim-)Hautdesinfektion, insbesondere im Rahmen von Katheterisierungen, vorzugsweise transurethralen oder suprapubischen Katheterisierungen, Endoskopien, Sondierungen, Intubationen und/oder geburtshilflichen Eingriffen.

Insbesondere betrifft die vorliegende Erfindung auch die Verwendung der erfindungsgemäßen Zusammensetzung, wie zuvor definiert, als Gleitmittel und/oder als Gleitgel insbesondere bei Katheterisierungen, insbesondere für einen Harnblasenkatheter, Sondierungen, Endoskopien, Intubationen und oder geburtshilflichen Eingriffen.

Demnach betrifft die vorliegende Erfindung auch die erfindungsgemäße Zusammensetzung als solche, wie zuvor definiert, zur Verwendung bei Katheterisierungen, Sondierungen, Endoskopien, Intubationen und oder geburtshilflichen Eingriffen, insbesondere als Gleitmittel und/oder als Gleitgel bei Katheterisierungen, Sondierungen, Endoskopien, Intubationen und oder geburtshilflichen Eingriffen, insbesondere für einen Harnblasenkatheter.

Die Verwendung der erfindungsgemäßen Zusammensetzung insbesondere auch bei der Katheterisierung ermöglicht nicht nur eine vergleichsweise schonende Katheterisierung und senkt das Risiko von Verletzungen bei der Katheterisierung, es wird vielmehr auch ein deutlich vermindertes Auftreten von Infektionen während der Verweildauer des Katheters beobachtet.

Zudem betrifft die vorliegende Erfindung die Verwendung der erfindungsgemäßen Zusammensetzung, wie zuvor definiert, zur kurativen und/oder prophylaktischen Behandlung von mit Katheterisierungen, Sondierungen, Endoskopien, Intubationen und oder geburtshilflichen Eingriffen einhergehenden oder hiermit im Zusammenhang stehenden Infektionen, Entzündungen und/oder Schmerzen, welche im Fall von Kathetisierungen insbesondere im Bereich des Harnleiters und/oder der Harnblase auftreten.

Folglich betrifft die vorliegende Erfindung auch die erfindungsgemäße Zusammensetzung als solche, wie zuvor definiert, zur Verwendung bei der kurativen und/oder prophylaktischen Behandlung von mit Katheterisierungen, Sondierungen, Endoskopien, Intubationen und oder geburtshilflichen Eingriffen einhergehenden oder hiermit im Zusammenhang stehenden Infektionen, Entzündungen und/oder Schmerzen, welche insbesondere im Fall von Kathetisierungen im Bereich des Harnleiters und/oder der Harnblase auftreten.

Im Allgemeinen kann die Zusammensetzung nach der Erfindung bei bzw. im Zusammenhang mit der Katheterisierung, insbesondere bei der Instillation eines Katheters, insbesondere eines Harnblasenkatheters, appliziert werden.

Als besonders vorteilhaft hat es sich erwiesen, wenn die Zusammensetzung bei der Katheterisierung, insbesondere vor der Instillation eines Katheters, insbesondere eines Harnblasenkatheters, in ein Körperlumen, insbesondere in die Harnröhre, appliziert wird. Üblicherweise ist es vorgesehen, dass die erfindungsgemäße Zusammensetzung vor Legen des Katheters in die Harnröhre instilliert wird, wobei es jedoch auch möglich ist, den Katheter zusätzlich während der Katheterisierung mit der erfindungsgemäßen Zusammensetzung zu benetzen.

Darüber hinaus ist es erfindungsgemäß möglich, dass die Zusammensetzung bei der Katheterisierung vor und/oder bei der Instillation eines Katheters, insbesondere eines Harnblasenkatheters, auf den Katheter aufgebracht wird. Hierbei kann es vorgesehen sein, dass die Zusammensetzung auf den in ein Körperlumen, insbesondere in eine Harnröhre, einführbaren und/oder einbringbaren Abschnitt eines Katheters aufgebracht wird.

Gegenstand der vorliegenden Erfindung ist insbesondere eine Zusammensetzung, wie sie zuvor beschrieben wurde, welche sich dadurch auszeichnet, dass die Zusammensetzung in einem Behältnis, insbesondere einer Applikationsvorrichtung, vorzugsweise in Form einer vorzugsweise sterilen Spritze, enthalten ist.

Mit anderen Worten betrifft die vorliegende Erfindung auch ein Behältnis, insbesondere eine Applikationsvorrichtung, vorzugsweise in Form einer vorzugsweise sterilen Spritze, welche die zuvor beschriebene erfindungsgemäße Zusammensetzung enthält.

Im Rahmen der vorliegenden Erfindung kann es dabei vorgesehen sein, dass das Behältnis zur Aufnahme einer Einmaldosis der Zusammensetzung ausgebildet ist.

Gemäß einer bevorzugten Ausführungsform der vorliegenden Erfindung weist das Behältnis ein zur Aufnahme der Zusammensetzung bestimmtes Volumen von 3 bis 30 cm³, insbesondere 3,5 bis 20 cm³, bevorzugt 4 bis 15 cm³, besonders bevorzugt 4,5 bis 12 cm³, insbesondere zur Aufnahme der erfindungsgemäßen Zusammensetzung, auf.

Gemäß einer besonderen Ausführungsform der vorliegenden Erfindung weist das Behältnis der Erfindung außerdem eine Applikationseinrichtung, insbesondere in Form eines Schlauches, zur Applikation bzw. Verabreichung der Zusammensetzung in einen Katheter, insbesondere einen Harnblasen- oder Nierenkatheter, aufnehmendes bzw. hieran angrenzendes Körperlumen, insbesondere zur Applikation und/oder Verabreichung der Zusammensetzung in die Harnröhre bzw. Harnblase, auf.

Gleichermaßen kann es im Rahmen der vorliegenden Erfindung vorgesehen sein, dass ein Katheter, insbesondere Harnblasenkatheter oder Fistelkatheter, mit der Zusammensetzung versehen ist, insbesondere wobei die Zusammensetzung, wie sie zuvor beschrieben wurde, auf einen in ein Körperlumen, insbesondere in die Harnröhre oder in eine künstliche Fistel zur Harnableitung, einführbaren und/oder einbringbaren Abschnitt des Katheters aufgebracht ist.

Mit anderen Worten betrifft die vorliegende Erfindung auch einen Katheter, insbesondere einen Harnblasenkatheter oder Fistelkatheter, wobei der Katheter mit der zuvor beschriebenen Zusammensetzung nach der Erfindung versehen ist bzw. diese aufweist. Hierbei kann es vorgesehen sein, dass die Zusammensetzung auch einen in ein Körperlumen, insbesondere in die Harnröhre, einführbare bzw. einbringbaren Abschnitt des Katheters aufgebracht ist.

Auch kann es erfindungsgemäß vorgesehen sein, dass die zuvor beschriebene Zusammensetzung in einer Verpackungseinheit vorliegt, welche mindestens ein Behältnis mit der erfindungsgemäßen Zusammensetzung, wie zuvor beschrieben, und/oder einen Katheter mit der erfindungsgemäßen Zusammensetzung, wie zuvor beschrieben, enthält.

Hierbei kann es vorgesehen sein, dass das Behältnis bzw. der Katheter in einer vor Kontamination schützenden Umverpackung eingebracht vorliegt.

Schließlich kann es im Hinblick auf die zuvor beschriebene Zusammensetzung auch vorgesehen sein, dass die Zusammensetzung in einem Kit, insbesondere Katheterisierungssystem, vorliegt, wobei das Kit als räumlich getrennte Komponenten einerseits einen Katheter, insbesondere einen Harnblasen- oder Fistelkatheter, und andererseits eine Zusammensetzung, wie sie zuvor beschrieben wurde, umfasst, insbesondere wobei die Zusammensetzung in ein Behältnis eingebracht ist.

Mit anderen Worten betrifft die vorliegende Erfindung auch ein Kit, insbesondere ein Katheterisierungssystem, umfassend als räumlich getrennte Komponenten einerseits einen Katheter, insbesondere einen Harnblasen- oder Fistelkatheter, und andererseits die zuvor beschriebene Zusammensetzung.

Hierbei kann es im Rahmen der vorliegenden Erfindung vorgesehen sein, dass die Zusammensetzung in ein erfindungsgemäßes Behältnis, wie zuvor definiert, eingebracht ist.

Das Kit zeichnet sich dadurch aus, dass die diesbezüglichen Komponenten bzw. Bestandteile zwar räumlich voneinander getrennt sind, aber funktional zusammenhängend vorliegen.

Weitere Ausgestaltungen, Abwandlungen und Variationen der vorliegenden Erfindung sind für den Fachmann beim Lesen der Beschreibung ohne Weiteres erkennbar und realisierbar ohne dass der dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen.

### Ausführungsbeispiele:

### 1. Bereitstellung von erfindungsgemäßen Zusammensetzungen sowie von Vergleichszusammensetzungen

Um die Wirksamkeit der erfindungsgemäßen Zusammensetzungen zu untersuchen, werden von der Anmelderin bereitgestellte erfindungsgemäße Zusammensetzungen mit verschiedenen Vergleichszusammensetzungen verglichen. Den nachfolgenden Tabellen 1 und 2 sind die Rezepturen für die bereitgestellten erfindungsgemäßen Zusammensetzungen A und B zu entnehmen.

**Tabelle 1: Rezeptur der erfindungsgemäßen Zusammensetzung A**

| **Komponente** | **Menge in Gew.-% (bezogen auf die Zusammensetzung)** |
|---|---|
| gereinigtes Wasser | 92-96 |
| Lidocainhydrochlorid | 1,5-3 |
| Polyhexanid (0,3 %) | 0,5-3 |
| Hydroxyethylcellulose | 0,5-2,5 |
| Natriumhydroxid-Lösung | 0,01-0,15 |

**Tabelle 2: Rezeptur der erfindungsgemäßen Zusammensetzung B**

| **Komponente** | **Menge in Gew.-% (bezogen auf die Zusammensetzung)** |
|---|---|
| gereinigtes Wasser | 92-96 |
| Benzocain hydrochlorid | 1,5-3 |
| Polyhexanid (0,3 %) | 0,5-3 |
| Hydroxyethylcellulose | 0,5-2,5 |
| Natriumhydroxid-Lösung | 0,01-0,15 |

Die Vergleichszusammensetzung C unterscheidet sich von der erfindungsgemäßen Zusammensetzung A dahingehend, dass anstelle des erfindungsgemäß eingesetzten Polyhexanids als Antiseptikum Octenidinhydrochlorid eingesetzt wird.

Die Vergleichszusammensetzung D unterscheidet sich dahingehend von der erfindungsgemäßen Zusammensetzung A, dass anstelle des erfindungsgemäß eingesetzten Polyhexanids als Antiseptikum Chlorhexidin eingesetzt wird.

Wie nachfolgend geschildert, werden die vier bereitgestellten Zusammensetzungen im Rahmen von Anwendungs- bzw. Wirksamkeitsstudien in Bezug auf ihre Anwendungseigenschaften und ihre Wirksamkeit bei Katheterisierungen untersucht.

### 2. Physikalische Eigenschaften / Stabilitätseigenschaften der erfindungsgemäßen Zusammensetzungen:

Nach ihrer Herstellung werden die erfindungsgemäßen Zusammensetzungen A und B sowohl vor als auch nach der Sterilisation in Bezug auf ihre physikalischen Eigenschaften untersucht. Die diesbezüglichen Ergebnisse sind den nachfolgenden Tabellen 3 bzw. 4 zu entnehmen.

**Tabelle 3: Eigenschaften der erfindungsgemäßen Zusammensetzung A**

| **Zusammensetzung A** | **vor Sterilisation** | **nach Sterilisation** |
|---|---|---|
| Aussehen | nahezu farblose, klare, viskose Flüssigkeit | nahezu farblose, klare, viskose Flüssigkeit |
| pH-Wert | 6,59 | 6,57 |
| Viskosität [mPa·s] (20 °C) | - | 2.764 |
| Gehalt Polyhexanid (mg/100 ml) | 1.567 | 1.531 |
| Gehalt Lidocainhydrochlorid (mg/100ml) | 2.031 | 2.000 |
| Unbekannte Verunreinigungen | nicht nachweisbar | nicht nachweisbar |

**Tabelle 4: Eiaenschaften der erfindunasaemäßen Zusammensetzung B**

| **Zusammensetzung B** | **vor Sterilisation** | **nach Sterilisation** |
|---|---|---|
| Aussehen | nahezu farblose, klare, viskose Flüssigkeit | nahezu farblose, klare, viskose Flüssigkeit |
| pH-Wert | 6,66 | 6,63 |
| Viskosität [mPa·s] (20 °C) | - | 2.770 |
| Gehalt Polyhexanid (mg/100 ml) | 1.543 | 1.510 |
| Gehalt Benzocainhydrochlorid (mg/100ml) | 2.021 | 2.016 |
| Unbekannte Verunreinigungen | nicht nachweisbar | nicht nachweisbar |

Wie die vorstehenden Ausführungen zeigen, zeichnen sich die erfindungsgemäßen Zusammensetzungen durch eine gute Stabilität aus. Auch nach erfolgter Sterilisation bleiben Aussehen, pH-Wert und Wirkstoffgehalt nahezu unverändert. Auch weisen die erfindungsgemäßen Zusammensetzungen nach erfolgter Sterilisation eine für die Anwendung optimale, da unveränderte Viskosität im erfindungsgemäß vorgesehenen Bereich auf.

Darüber hinaus werden die erfindungsgemäßen Zusammensetzungen in Bezug auf ihre Langzeitstabilität, d. h. ihre Stabilität über einen Zeitraum von zwei Jahren, untersucht. Hier zeigt sich, dass auch nach einer Lagerung über einen Zeitraum von zwei Jahren die optischen Eigenschaften unverändert bleiben, insbesondere keine Eintrübung oder Kristallisation zu beobachten ist. Darüber hinaus bleiben auch die Wirkstoffgehalte zumindest im Wesentlichen konstant.

Auch hat sich im Rahmen der Untersuchung der Langzeitstabilität gezeigt, dass die erfindungsgemäßen Zusammensetzungen keinen mikrobiologischen Befall bzw. keine bakteriellen Kontaminationen aufweisen, obwohl die Zusammensetzungen nach der vorliegenden Erfindung frei von Konservierungsmitteln sind. Insgesamt besitzen die erfindungsgemäßen Zusammensetzungen somit eine hervorragende Langzeit- bzw. Lagerstabilität.

### 3. Anwendungs- und Wirksamkeitsstudien:

Im Rahmen von seitens der Anmelderin durchgeführten Wirksamkeitsstudien wird die herausragende Wirkung von Zusammensetzungen nach der vorliegenden Erfindung auf Basis der erfindungsgemäßen Wirkstoffkombination von Polyhexanid einerseits und einem Lokalanästhetikum andererseits (vgl. nachfolgende "Zusammensetzung A" (Lokalanästhetikum: Lidocain) und "Zusammensetzung B" (Lokalanästhetikum: Benzocain)) gegenüber Vergleichszusammensetzungen auf Basis von Octenidin und Lidocain als Wirkstoffkombination (vgl. "Zusammensetzung C") sowie auf Basis von Chlorhexidin und Lidocain (vgl. nachfolgende "Zusammensetzung D") belegt. In diesem Zusammenhang wird für die erfindungsgemäße Kombination auf Basis der nachfolgenden Zusammensetzung A bzw. Zusammensetzung B eine deutlich verbesserte Wirksamkeit sowohl hinsichtlich der Verminderung von Infektionen als auch im Hinblick auf die schmerzstillenden Eigenschaften beobachtet.

Die durchgeführte Wirksamkeitsstudie wird anhand von verschiedenen Untersuchungsmethoden (d.h. subjektives Wohlbefinden der Patienten, Erreger- bzw. Bakteriengehalt in einer Probe des Probanden nach Entfernung des Katheters) ermittelt.

Im Rahmen der nachfolgend ausgeführten Untersuchungen werden den Probanden vor dem Setzen des Katheters jeweils entsprechende Katheterisierungsgele auf Basis der obigen Zusammensetzungen in den Harnleiter appliziert und nachfolgend die entsprechenden Katheter gesetzt. Pro Untersuchungsgruppe bzw. Zusammensetzung werden jeweils zehn Probanden untersucht, wobei die Probanden über die Gruppen verteilt ein Alter von 40 bis 69 Jahren aufweisen. Jede Untersuchungsgruppe besteht dabei aus 5 männlichen und 5 weiblichen Probanden.
a) Im Rahmen des ersten Untersuchungskomplexes führen die Probanden ihr subjektives Empfinden anhand eines Schulnotensystems mit Noten von 1 bis 6 (1= sehr gut bis 6 = ungenügend) an. Bei dieser Untersuchung wird insbesondere auf Schmerzen beim Setzen des Katheters sowie auf das Schmerzempfinden zum Zeitpunkt t = 6 Stunden, 12 Stunden bzw. 24 Stunden nach erfolgter Katheterisierung abgestellt.
Mit den erfindungsgemäßen Zusammensetzungen A und B kann eine deutliche Verbesserung der Schmerzsymptomatik beobachtet werden. Sowohl beim Setzen des Katheters als auch zu den späteren Zeitpunkten nach erfolgter Katheterisierung kann die Schmerzsymptomatik bei den Probanden signifikant verbessert werden. Der Einsatz von Lidocain als Lokalanästhetikum erweist sich dabei als leicht überlegen gegenüber dem Einsatz von Benzocain. Dennoch lässt sich insgesamt auch mit dem Einsatz von Benzocain eine hervorragende Schmerzlinderung erzielen. Mit den Vergleichszusammensetzungen C und D werden schlechtere Ergebnisse erzielt. Die ermittelten Ergebnisse für alle Gruppen sind der nachfolgenden Tabelle 5 zu entnehmen:

**Tabelle 5: Ergebnisse der Beurteilung der Schmerzsymptomatik**

| | **A (Erfindung)** | **B (Erfindung)** | **C (Vergleich)** | **D (Vergleich)** |
|---|---|---|---|---|
| t = 0 h | 1,2 ± 0,1 | 1,3 ± 0,2 | 1,6 ± 0,4 | 2,0 ± 0,3 |
| t = 6 h | 1,4 ± 0,3 | 1,5 ± 0,1 | 1,9 ± 0,5 | 2,6 ± 0,1 |
| t = 12 h | 1,7 ± 0,1 | 1,8 ± 0,3 | 2,2 ± 0,3 | 2,9 ± 0,5 |
| t = 24 h | 2,0 ± 0,2 | 2,1 ± 0,2 | 2,4 ± 0,6 | 3,3 ± 0,2 |

b) In einem zweiten Untersuchungskomplex wird der Katheter nach 72 Stunden entfernt und nachfolgend eine Probe der jeweiligen Probanden im Hinblick auf einen etwaigen mikrobiologischen Befall untersucht. Die Klassifizierung erfolgt gleichermaßen auf Basis des zuvor beschriebenen Schulnotensystems mit Noten von 1 bis 6 (1 = sehr geringer bzw. kein mikrobiologischer Befall bis 6 = sehr starker mikrobiologischer Befall der gewonnen Probe). Es werden entsprechende Mittelwerte sowie die dazugehörigen Standardabweichungen ermittelt. Die nachfolgende Tabelle 6 zeigt die ermittelten Ergebnisse für alle Gruppen. In Bezug auf die erfindungsgemäßen Zusammensetzungen A und B liegt eine signifikante Reduktion des mikrobiologischen Befalls vor. Mit den Vergleichszusammensetzungen C und D, insbesondere Zusammensetzung D, werden schlechtere Ergebnisse erzielt.

**Tabelle 6: Ergebnisse der Beurteilung des mikrobiologischen Befalls**

| | **A (Erfindung)** | **B (Erfindung)** | **C (Vergleich)** | **D (Vergleich)** |
|---|---|---|---|---|
| t = 72 h | 1,1 ± 0,2 | 1,2 ± 0,1 | 1,4 ± 0,1 | 2,9 ± 0,4 |

c) In einem dritten Untersuchungskomplex wird beurteilt, inwieweit die jeweils getesteten Zusammensetzungen Hautirritationen bzw. Unverträglichkeiten auslösen. Auch in diesem Zusammenhang erfolgt die Klassifizierung auf Basis der zuvor genannten Schulnotenskala von 1 bis 6 (1 = keine Irritation bzw. Unverträglichkeiten bis 6 = sehr starke Unverträglichkeiten bzw. Irritationen). Es werden die entsprechenden Mittelwerte sowie die dazugehörigen Standardabweichungen ermittelt. Die nachfolgende Tabelle 7 zeigt die diesbezüglich ermittelten Ergebnisse für sämtliche Gruppen. In Bezug auf die erfindungsgemäßen Zusammensetzungen A und B werden keinerlei Unverträglichkeiten beobachtet. Die Vergleichszusammensetzungen C und D werden im Mittel weniger gut von den Probanden vertragen.

**Tabelle 7: Beurteilung der Verträglichkeit**

| **A (Erfindung)** | **B (Erfindung)** | **C (Vergleich)** | **D (Vergleich)** |
|---|---|---|---|
| 1,2 ± 0,2 | 1,3 ± 0,1 | 2,3 ± 0,3 | 3,2 ± 0,4 |

Die vorstehenden Versuchsreihen zeigen insgesamt die hervorragende Wirksamkeit der erfindungsgemäßen Zusammensetzungen auf Basis von Polyhexanid einerseits und einem Lokalanästhetikum, insbesondere Lidocainhydrochlorid, andererseits gegenüber den angeführten Vergleichszusammensetzungen. Insbesondere hat sich im Rahmen der vorliegenden Erfindung gezeigt, dass durch den Einsatz von Polyhexanid als Antiseptikum die Verträglichkeit von Gleitmitteln bzw. Gleitgelen signifikant verbessert werden kann. Darüber hinaus werden eine hervorragende antimikrobielle Wirkung und eine effiziente Schmerzlinderung erzielt.

Zudem weisen die erfindungsgemäßen Zusammensetzungen eine hervorragende (Lager-)Stabilität auf. Auch über einen Lagerungszeitraum von zwei Jahren treten keine relevanten Veränderungen der physikalischen Eigenschaften auf. Was zudem speziell die mikrobielle Stabilität der erfindungsgemäßen Zusammensetzungen anbelangt, so wird diese auch ohne den Einsatz eines zusätzlichen Konservierungsmittels gewährleistet.

## Patentansprüche

1. Zusammensetzung zur Verwendung bei der prophylaktischen und/oder therapeutischen und/oder diagnostischen Schleimhautdesinfektion im Rahmen von transurethralen oder suprapubischen Katheterisierungen, Sondierungen, Endoskopien, Intubationen und/oder geburtshilflichen Eingriffen, wobei die Zusammensetzung in Form eines Gleitgels und/oder Gleitmittels vorliegt,
wobei die Zusammensetzung jeweils in wirksamen, insbesondere pharmazeutisch wirksamen Mengen
(a) mindestens ein Lokalanästhetikum als "Komponente (a)" und
(b) Polyhexanid und/oder dessen Salze als "Komponente (b)", insbesondere als antiseptischen Wirk- und/oder Inhaltsstoff (Desinfektionsmittel),
enthält,
wobei die Zusammensetzung frei ist von Polyalkylenglykolen und Alkylenglykolen undwobei die Zusammensetzung einen physiologisch verträglichen pH-Wert im Bereich von 5 bis 7,5 aufweist.

2. Zusammensetzung zur Verwendung nach Anspruch 1,
wobei die Komponente (a) ausgewählt ist aus der Gruppe von Lokalanästhetika vom Estertyp und Lokalanästhetika vom Amidtyp, insbesondere aus der Gruppe von Benzocain, Procain, Tetracain, Lidocain, Etidocain, Prilocain, Mepivacain, Bupivacain und S-Ropivacain oder deren Salzen sowie deren Kombinationen oder Mischungen, vorzugsweise Lidocain und/oder dessen Salzen; und/oder
wobei die Zusammensetzung als Komponente (a) Lidocain und/oder dessen Salze, insbesondere Lidocainhydrochlorid, enthält; und/oder
wobei die Zusammensetzung die Komponente (a) in einer Menge im Bereich von 0,1 bis 10 Gew.-%, insbesondere im Bereich von 0,5 bis 8 Gew.-%, vorzugsweise im Bereich von 0,7 bis 6 Gew.-%, bevorzugt im Bereich von 1 bis 3 Gew.-%, bezogen auf die Zusammensetzung, enthält; und/oder
wobei die Zusammensetzung die Komponente (a) in einer Menge von mindestens 0,01 Gew.-%, insbesondere mindestens 0,1 Gew.-%, vorzugsweise mindestens 0,5 Gew.-%, bevorzugt mindestens 1 Gew.-%, bezogen auf die Zusammensetzung, enthält und/oder wobei die Zusammensetzung die Komponente (a) in einer Menge von höchstens 15 Gew.-%, insbesondere höchstens 10 Gew.-%, vorzugsweise höchstens 8 Gew.-%, bevorzugt höchstens 5 Gew.-%, besonders bevorzugt höchstens 3 Gew.-%, bezogen auf die Zusammensetzung, enthält.

3. Zusammensetzung zur Verwendung nach Anspruch 1 oder 2,
wobei die Zusammensetzung Polyhexanid und/oder dessen Salze in einer Menge im Bereich von 0,05 bis 10 Gew.-%, insbesondere im Bereich von 0,1 bis 8 Gew.-%, vorzugsweise im Bereich von 0,5 bis 5 Gew.-%, bevorzugt im Bereich von 0,75 bis 3 Gew.-%, besonders bevorzugt im Bereich von 1 bis 2 Gew.-%, bezogen auf die Zusammensetzung, enthält; und/oder
wobei die Zusammensetzung Polyhexanid und/oder dessen Salze in einer Menge von mindestens 0,05 Gew.-%, insbesondere mindestens 0,1 Gew.-%, vorzugsweise mindestens 0,5 Gew.-%, bevorzugt mindestens 0,75 Gew.-%, besonders bevorzugt mindestens 1 Gew.-%, bezogen auf die Zusammensetzung, enthält, und/oder wobei die Zusammensetzung das Polyhexanid in einer Menge von höchstens 10 Gew.-%, insbesondere höchstens 8 Gew.-%, vorzugsweise höchstens 5 Gew.-%, bevorzugt höchstens 3 Gew.-%, besonders bevorzugt höchstens 2 Gew.-%, bezogen auf die Zusammensetzung, enthält.

4. Zusammensetzung zur Verwendung nach einem der vorangehenden Ansprüche,
wobei die Zusammensetzung die Komponente (a) und die Komponente (b) in einem gewichtsbezogenen Verhältnis von [(a) : (b)] im Bereich von 1 : 50 bis 50 : 1, insbesondere im Bereich von 1 : 20 bis 20 : 1, vorzugsweise im Bereich von 1 : 10 bis 10 : 1, bevorzugt im Bereich von 1 : 5 bis 5 : 1, besonders bevorzugt 1 : 3 bis 3 : 1, enthält; und/oder
wobei die Zusammensetzung wässrig oder wässrig-alkoholisch basiert ist; und/oder
wobei die Zusammensetzung Wasser, insbesondere gereinigtes Wasser, in einer Menge im Bereich von 30 bis 99 Gew.-%, insbesondere im Bereich von 40 bis 98 Gew.-%, bevorzugt im Bereich von 50 bis 97 Gew.-%, besonders bevorzugt im Bereich von 60 bis 96 Gew.-%, bezogen auf die Zusammensetzung, enthält.

5. Zusammensetzung zur Verwendung nach einem der vorangehenden Ansprüche,
wobei die Zusammensetzung mindestens einen vorzugsweise mehrwertigen Alkohol, insbesondere ausgewählt aus der Gruppe von Polyvinylalkoholen, Glycerin sowie deren Kombinationen, vorzugsweise Glycerin, enthält und/oder wobei die Zusammensetzung zumindest im Wesentlichen kein Propylenglykol aufweist, insbesondere wobei die Zusammensetzung den vorzugsweise mehrwertigen Alkohol in einer Menge im Bereich von 1 bis 80 Gew.-%, insbesondere im Bereich von 5 bis 70 Gew.-%, vorzugsweise im Bereich von 10 bis 60 Gew.-%, bevorzugt im Bereich von 15 bis 50 Gew.-%, bezogen auf die Zusammensetzung, enthält; und/oder
wobei die Zusammensetzung eine Mischung oder ein Gemisch von Wasser und mindestens einem vorzugsweise mehrwertigen Alkohol aufweist, insbesondere wobei der Anteil an Wasser mindestens 10 Gew.-%, insbesondere mindestens 30 Gew.-%, bevorzugt mindestens 50 Gew.-%, bezogen auf die Mischung oder das Gemisch, beträgt und/oder insbesondere wobei das gewichtsbezogene Verhältnis von Wasser zu Alkohol in der Zusammensetzung im Bereich von 9 : 1 bis 1 : 5, insbesondere im Bereich von 3 : 1 bis 1 : 3, vorzugsweise im Bereich von 2 : 1 bis 1 : 2, bevorzugt im Bereich von 1,5 : 1 bis 1 : 1,5, liegt.

6. Zusammensetzung zur Verwendung nach einem der vorangehenden Ansprüche, wobei die Zusammensetzung mindestens einen Gelbildner aufweist, insbesondere wobei der Gelbildner ausgewählt ist aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen, vorzugsweise Cellulose und Cellulosederivaten, bevorzugt modifizierten Cellulosen, besonders bevorzugt chemisch modifizierten Cellulosen, ganz besonders bevorzugt Methylcellulose, Carboxymethylcellulose und/oder Hydroxyethylcellulose, noch mehr bevorzugt Hydroxyethylcellulose, insbesondere wobei die Zusammensetzung den Gelbildner in einer Menge im Bereich von 0,01 bis 20 Gew.-%, insbesondere im Bereich von 0,1 bis 10 Gew.-%, vorzugsweise im Bereich von 0,5 bis 5 Gew.-%, bevorzugt im Bereich von 1 bis 3 Gew.-%, bezogen auf die Zusammensetzung, aufweist.

7. Zusammensetzung zur Verwendung nach einem der vorangehenden Ansprüche,
wobei die Zusammensetzung bei einer Temperatur von 20 °C eine dynamische Viskosität im Bereich von 20 bis 20.000 mPa·s, insbesondere im Bereich von 50 bis 15.000 mPa·s, vorzugsweise im Bereich von 100 bis 10.000 mPa·s, bevorzugt im Bereich von 500 bis 8.000 mPa·s, besonders bevorzugt im Bereich von 1.000 bis 7.000 mPa·s, noch mehr bevorzugt im Bereich von 2.000 bis 6.000 mPa·s, aufweist; und/oder
wobei die Zusammensetzung einen physiologisch verträglichen pH-Wert im Bereich von 6 bis 7 aufweist; und/oder
wobei die Zusammensetzung eine physiologisch verträgliche elektrische Leitfähigkeit, insbesondere im Bereich von 1 bis 20 mS/cm, vorzugsweise im Bereich von 2 bis 15 mS/cm, bevorzugt im Bereich von 3 bis 13 mS/cm, besonders bevorzugt im Bereich von 5 zu 10 mS/cm, aufweist.

8. Zusammensetzung zur Verwendung nach einem der vorangehenden Ansprüche,
wobei die Zusammensetzung mindestens eine Säure und/oder Base, insbesondere zur Ausbildung eines Puffersystems, aufweist und/oder wobei die Zusammensetzung mindestens eine Base aufweist, insbesondere wobei die Base ausgewählt ist aus der Gruppe von Aminen, Carboxylaten, Alkali- und/oder Erdalkalimetallhydroxiden sowie deren Kombinationen, insbesondere Alkali- und Erdalkalimetallhydroxyden, bevorzugt Natriumhydroxid; und/oder
wobei die Zusammensetzung kein Konservierungsmittel und/oder keinen Konservierungsstoff aufweist und/oder wobei die Zusammensetzung zumindest im Wesentlichen frei ist von Konservierungsmitteln und/oder Konservierungsstoffen.

9. Zusammensetzung zur Verwendung nach einem der vorangehenden Ansprüche,
wobei die Zusammensetzung mindestens einen weiteren Wirk- und/oder Inhaltsstoff, insbesondere ausgewählt aus der Gruppe von Hautschutzmitteln, Antiseptika, Lokalanästhetika, Vitaminen, Spurenelementen, Mineralien, Mikronährstoffen sowie deren Kombinationen, enthält; und/oder
wobei die Zusammensetzung mindestens einen üblichen pharmazeutischen Zusatz- und/oder Hilfsstoff enthält, insbesondere ausgewählt aus der Gruppe von Verarbeitungshilfsstoffen, Stabilisatoren, Emulgatoren, Antioxidantien, Konservierungsstoffen, Feuchthaltemitteln, pH-Stellmitteln, pH-Puffersubstanzen, Verdickungsmitteln, Antiseptika, Färbe-, Puffer-, Riech-, Duft-, Streck-, Binde-, Netz- und/oder Konservierungsstoffen sowie deren Kombinationen.

10. Zusammensetzung zur Verwendung nach einem der vorangehenden Ansprüche, **dadurch gekennzeichnet, dass** die Zusammensetzung in einem Behältnis, insbesondere einer Applikationsvorrichtung, vorzugsweise in Form einer vorzugsweise sterilen Spritze, enthalten ist.

11. Zusammensetzung zur Verwendung nach einem der vorangehenden Ansprüche, **dadurch gekennzeichnet, dass** ein Katheter, insbesondere Harnblasenkatheter oder Fistelkatheter, mit der Zusammensetzung versehen ist, insbesondere wobei die Zusammensetzung auf einen in ein Körperlumen, insbesondere in die Harnröhre oder in eine künstliche Fistel zur Harnableitung, einführbaren und/oder einbringbaren Abschnitt des Katheters aufgebracht ist.

12. Zusammensetzung zur Verwendung nach einem der vorangehenden Ansprüche, **dadurch gekennzeichnet, dass** die Zusammensetzung in einem Kit, insbesondere Katheterisierungssystem, vorliegt, wobei das Kit als räumlich getrennte Komponenten einerseits einen Katheter, insbesondere einen Harnblasen- oder Fistelkatheter, und andererseits eine Zusammensetzung zur Verwendung nach einem der vorangehenden Ansprüche umfasst, insbesondere wobei die Zusammensetzung in ein Behältnis eingebracht ist.

## Claims

1. Composition for use in the prophylactic and/or therapeutic and/or diagnostic mucous membrane disinfection as part of transurethral or suprapubic catheterisations, probes, endoscopies, intubations and/or obstetric interventions, wherein the composition exists in the form of a lubricating gel and/or lubricating agent,
wherein the composition contains in effective, particularly pharmaceutically active quantities
(a) at least one local anaesthetic as "Component (a)" and
(b) polyhexanide and/or its salts as "Component (b)", particularly as an antiseptic active substance and/or ingredient (disinfectant),
wherein the composition is free from polyalkylene glycols and alkylene glycols and wherein the composition has a physiologically compatible pH value in the range of 5 to 7.5.

2. The composition for use according to claim 1,
wherein Component (a) is selected from the group of local anaesthetics of ester type and local anaesthetics of amide type, particularly from the group of benzocaine, procaine, tetracaine, lidocaine, etidocaine, prilocaine, mepivacaine, bupivacaine and S-ropivacaine or their salts and their combinations or mixtures, preferably lidocaine and/or its salts; and/or
wherein the composition contains as Component (a) lidocaine and/or its salts, particularly lidocaine hydrochloride; and/or
wherein the composition contains Component (a) in a quantity in the range of 0.1 to 10 wt.-%, particularly in the range of 0.5 to 8 wt.-%, preferably in the range of 0.7 to 6 wt.-%, more preferably in the range of 1 to 3 wt.-%, in relation to the composition; and/or
wherein the composition contains Component (a) in a quantity of at least 0.01 wt.-%, particularly at least 0.1 wt.-%, preferably at least 0.5 wt.-%, more preferably at least 1 wt.-%, in relation to the composition, and/or wherein the composition contains Component (a) in a quantity of at most 15 wt.-%, particularly at most 10 wt.-%, preferably at most 8 wt.-%, more preferably at most 5 wt.-%, particularly preferably at most 3 wt.-%, in relation to the composition.

3. The composition for use according to claim 1 or 2,
wherein the composition contains polyhexanide and/or its salts in a quantity in the range of 0.05 to 10 wt.-%, particularly in the range of 0.1 to 8 wt.-%, preferably in the range of 0.5 to 5 wt.-%, more preferably in the range of 0.75 to 3 wt.-%, particularly preferably in the range of 1 to 2 wt.-%, in relation to the composition; and/or
wherein the composition contains polyhexanide and/or its salts in a quantity of at least 0.05 wt.-%, particularly at least 0.1 wt.-%, preferably at least 0.5 wt.-%, more preferably at least 0.75 wt.-%, particularly preferably at least 1 wt.-%, in relation to the composition, and/or wherein the composition contains polyhexanide in a quantity of at most 10 wt.-%, particularly at most 8 wt.-%, preferably at most 5 wt.-%, more preferably at most 3 wt.-%, particularly preferably at most 2 wt.-%, in relation to the composition.

4. The composition for use according to one of the preceding claims,
wherein the composition contains Component (a) and Component (b) in a weight-related ratio of [(a) : (b)] in the range of 1 : 50 to 50 : 1, particularly in the range of 1 : 20 to 20 : 1, preferably in the range of 1 : 10 to 10 : 1, more preferably in the range of 1 : 5 to 5 : 1, particularly preferably 1 : 3 to 3 : 1; and/or
wherein the composition has an aqueous or hydroalcoholic basis; and/or
wherein the composition contains water, particularly purified water, in a volume in the range of 30 to 99 wt.-%, particularly in the range of 40 to 98 wt.-%, more preferably in the range of 50 to 97 wt.-%, particularly preferably in the range of 60 to 96 wt.-%, in relation to the composition.

5. The composition for use according to one of the preceding claims,
wherein the composition contains at least one preferably polyvalent alcohol, particularly selected from the group of polyvinyl alcohols, glycerine and its combinations, preferably glycerine, and/or wherein the composition has at least substantially no propylene glycol, particularly wherein the composition contains the preferably polyvalent alcohol in a quantity in the range of 1 to 80 wt.-%, particularly in the range of 5 to 70 wt.-%, preferably in the range of 10 to 60 wt.-%, more preferably in the range of 15 to 50 wt.-%, in relation to the composition; and/or
wherein the composition has a mixture or a compound of water and at least one preferably polyvalent alcohol, particularly wherein the proportion of water is at least 10 wt.-%, particularly at least 30 wt.-%, more preferably at least 50 wt.-%, in relation to the mixture or compound, and/or particularly wherein the weight-related ratio of water to alcohol in the composition is in the range of 9 : 1 to 1 : 5, particularly in the range of 3 : 1 to 1 : 3, preferably in the range of 2 : 1 to 1 : 2, more preferably in the range of 1.5 : 1 to 1 : 1.5.

6. The composition for use according to one of the preceding claims, wherein the composition has at least one gel forming agent, particularly wherein the gel forming agent is selected from the group of bentonites, silicic acids, polyacrylic acids, carbomers, polyvinylpyrrolidones, cellulose and cellulose derivatives, xanthanes and their mixtures, preferably cellulose and cellulose derivatives, more preferably modified celluloses, particularly preferably chemically modified celluloses, very particularly preferably methylcellulose, carboxymethyl cellulose and/or hydroxyethyl cellulose, even more preferably hydroxyethyl cellulose, particularly wherein the composition has the gel forming agent in a quantity in the range of 0.01 to 20 wt.-%, particularly in the range of 0.1 to 10 wt.-%, preferably in the range of 0.5 to 5 wt.-%, more preferably in the range of 1 to 3 wt.-%, in relation to the composition.

7. The composition for use according to one of the preceding claims,
wherein the composition has at a temperature of 20 °C a dynamic viscosity in the range of 20 to 20,000 mPa·s, particularly in the range of 50 to 15,000 mPa·s, preferably in the range of 100 to 10,000 mPa·s, more preferably in the range of 500 to 8,000 mPa·s particularly preferably in the range of 1,000 to 7,000 mPa·s, even more preferably in the range of 2,000 to 6,000 mPa·s; and/or
wherein the composition has a physiologically compatible pH value in the range of 6 to 7; and/or
wherein the composition has a physiologically compatible electrical conductivity, particularly in the range of 1 to 20 mS/cm, preferably in the range of 2 to 15 mS/cm, more preferably in the range of 3 to 13 mS/cm, particularly preferably in the range of 5 to 10 mS/cm.

8. The composition for use according to one of the preceding claims,
wherein the composition has at least one acid and/or base, particularly for the formation of a buffer system, and/or wherein the composition has at least one base, particularly wherein the base is selected from the group of amines, carboxylates, alkali and/or alkaline earth metal hydroxides and their combinations, particularly alkali and alkaline earth metal hydroxides, more preferably sodium hydroxide; and/or
wherein the composition has no preservative and/or no preserving agent and/or wherein
the composition is at least substantially free from preservatives and/or preserving agents.

9. The composition for use according to one of the preceding claims,
wherein the composition contains at least one further active agent and/or ingredient, particularly chosen from the group of skin protection products, antiseptics, local anaesthetics, vitamins, trace elements, minerals, micronutrients and their combinations; and/or
wherein the composition contains at least one customary pharmaceutical additive and/or auxiliary agent, particularly chosen from the group of processing aids, stabilisers, emulsifiers, antioxidants, preservatives, humectants, pH suspending agents, pH buffer substances, thickening agents, antiseptics, colourants, buffer substances, aromatic substances, fragrances, diluting agents, binders, netting agents and/or preserving agents and their combinations.

10. The composition for use according to one of the preceding claims, **characterised in that** the composition is contained in a container, particularly an application device, preferably in the form of a preferably sterile syringe.

11. The composition for use according to one of the preceding claims, **characterised in that** a catheter, particularly a urinary bladder catheter or fistula catheter, is provided with the composition, particularly wherein the composition is applied to a section of the catheter that can be inserted and/or can be introduced into a body lumen, particularly into the urethra or into an artificial fistula for urinary diversion.

12. The composition for use according to one of the preceding claims, **characterised in that** the composition exists in a kit, particularly a catheterization system, wherein the kit comprises as physically separate components a catheter, particularly a urinary bladder or fistula catheter, and a composition for use according to one of the preceding claims, particularly wherein the composition is placed in a container.

## Revendications

1. Composition destinée à être utilisée pour la désinfection prophylactique et/ou thérapeutique et/ou diagnostique des muqueuses, dans le cadre de la pose de cathéters ou de sondes, des endoscopies, des intubations et/ou des interventions obstétriques transurétrales ou suspubiennes, la composition se présentant sous la forme d'un gel et/ou d'un agent lubrifiant,
la composition contenant, chacun en quantités actives, en particulier pharmaceutiquement actives,
(a) au moins un anesthésique local comme « constituant (a) » et
(b) du polyhexanide et/ou l'un de ses sels comme « constituant (b) », en particulier en tant que principe actif et/ou ingrédient antiseptique (agent désinfectant),
la composition étant exempte de polyalkylèneglycols et d'alkylèneglycols et la composition ayant une valeur de pH physiologiquement acceptable située dans la plage allant de 5 à 7,5.

2. Composition destinée à être utilisée selon la revendication 1,
dans laquelle le constituant (a) est choisi dans le groupe constitué par des anesthésiques locaux de type ester et des anesthésiques locaux de type amide, en particulier dans le groupe constitué par la benzocaïne, la procaïne, la tétracaïne, la lidocaïne, l'étidocaïne, la prilocaïne, la mépivacaïne, la bupivacaïne et la S-ropivacaïne ou l'un de leurs sels ainsi que des combinaisons ou des mélanges de ceux-ci, de préférence la lidocaïne et/ou l'un de ses sels ; et/ou
la composition contenant, comme constituant (a), la lidocaïne et/ou l'un de ses sels, en particulier le chlorhydrate de lidocaïne ; et/ou
la composition contenant le constituant (a) dans une proportion située dans la plage allant de 0,1 à 10 % en poids, en particulier dans la plage allant de 0,5 à 8 % en poids, de préférence dans la plage allant de 0,7 à 6 % en poids, de manière davantage préférée dans la plage allant de 1 à 3 % en poids, par rapport à la composition ; et/ou
la composition contenant le constituant (a) dans une proportion supérieure ou égale à 0,01 % en poids, en particulier supérieure ou égale à 0,1 % en poids, de préférence supérieure ou égale à 0,5 % en poids, de manière davantage préférée supérieure ou égale à 1 % en poids, par rapport à la composition et/ou la composition contenant le constituant (a) dans une proportion inférieure ou égale à 15 % en poids, en particulier inférieure ou égale à 10 % en poids, de préférence inférieure ou égale à 8 % en poids, de manière davantage préférée inférieure ou égale à 5 % en poids, de manière particulièrement préférée inférieure ou égale à 3 % en poids par rapport à la composition.

3. Composition destinée à être utilisée selon la revendication 1 ou 2,
la composition contenant le polyhexanide et/ou l'un de ses sels dans une proportion située dans la plage allant de 0,05 à 10 % en poids, en particulier dans la plage allant de 0,1 à 8 % en poids, de préférence dans la plage allant de 0,5 à 5 % en poids, de manière davantage préférée dans la plage allant de 0,75 à 3 % en poids, de manière particulièrement préférée dans la plage allant de 1 à 2 % en poids par rapport à la composition ; et/ou
la composition contenant le polyhexanide et/ou l'un de ses sels dans une proportion supérieure ou égale à 0,05 % en poids, en particulier supérieure ou égale à 0,1 % en poids, de préférence supérieure ou égale à 0,5 % en poids, de manière davantage préférée supérieure ou égale à 0,75 % en poids, de manière particulièrement préférée supérieure ou égale à 1 % en poids par rapport à la composition, et/ou la composition contenant le polyhexanide dans une proportion inférieure ou égale à 10 % en poids, en particulier inférieure ou égale à 8 % en poids, de préférence inférieure ou égale à 5 % en poids, de manière davantage préférée inférieure ou égale à 3 % en poids, de manière particulièrement préférée inférieure ou égale à 2 % en poids par rapport à la composition.

4. Composition destinée à être utilisée selon l'une des revendications précédentes,
la composition contenant le constituant (a) et le constituant (b) dans un rapport pondéral [(a):(b)] situé dans la plage allant de 1:50 à 50:1, en particulier situé dans la plage allant de 1:20 à 20:1, de préférence situé dans la plage allant de 1:10 à 10:1, de manière davantage préférée situé dans la plage allant de 1:5 à 5:1, de manière particulièrement préférée situé dans la plage allant de 1:3 à 3:1 ; et/ou
la composition étant à base d'eau ou d'alcool et d'eau ; et/ou
la composition contenant de l'eau, en particulier de l'eau purifiée, dans une proportion située dans la plage allant de 30 à 99 % en poids, en particulier dans la plage allant de 40 à 98 % en poids, de préférence dans la plage allant de 50 à 97 % en poids, de manière particulièrement préférée dans la plage allant de 60 à 96 % en poids par rapport à la composition.

5. Composition destinée à être utilisée selon l'une des revendications précédentes,
la composition contenant au moins un alcool, de préférence un polyalcool, choisi en particulier dans le groupe constitué par des alcools polyvinyliques, de la glycérine ainsi que des combinaisons de ceux-ci, de préférence de la glycérine et/ou la composition ne contenant au moins pratiquement pas de propylèneglycol, en particulier la composition contenant de préférence le polyalcool dans une proportion située dans la plage allant de 1 à 80 % en poids, en particulier dans la plage allant de 5 à 70 % en poids, de préférence dans la plage allant de 10 à 60 % en poids, de manière davantage préférée dans la plage allant de 15 à 50 % en poids, par rapport à la composition ; et/ou
la composition contenant un mélange ou une combinaison d'eau et de préférence au moins un polyalcool, la proportion d'eau étant en particulier supérieure ou égale à 10 % en poids, en particulier supérieure ou égale à 30 % en poids, de préférence supérieure ou égale à 50 % en poids, par rapport au mélange ou à la combinaison, et/ou le rapport pondéral entre l'eau et l'alcool dans la composition étant situé dans la plage allant de 9:1 à 1:5, en particulier situé dans la plage allant de 3:1 à 1:3, de préférence situé dans la plage allant de 2:1 à 1:2, de manière davantage préférée situé dans la plage allant de 1,5:1 à 1:1,5.

6. Composition destinée à être utilisée selon l'une des revendications précédentes, la composition contenant au moins un agent gélifiant, l'agent gélifiant étant en particulier choisi dans le groupe constitué par des bentonites, des acides siliciques, des acides polyacryliques, des carbomères, des polyvinylpyrrolidones, de la cellulose et des dérivés de cellulose, des xanthanes ainsi que des mélanges de ceux-ci, en particulier de la cellulose et des dérivés de cellulose, de préférence des celluloses modifiées, de manière particulièrement préférée des celluloses modifiées chimiquement, de manière particulièrement préférée entre toutes de la méthylcellulose, de la carboxyméthylcellulose et/ou de l'hydroxyéthylcellulose, de manière encore davantage préférée de l'hydroxyéthylcellulose, en particulier la composition contenant l'agent gélifiant dans une proportion située dans la plage allant de 0,01 à 20 % en poids, en particulier dans la plage allant de 0,1 à 10 % en poids, de préférence dans la plage allant de 0,5 à 5 % en poids, de manière davantage préférée dans la plage allant de 1 à 3 % en poids, par rapport à la composition.

7. Composition destinée à être utilisée selon l'une des revendications précédentes,
la composition présentant, à une température de 20 °C, une viscosité dynamique située dans la plage allant de 20 à 20 000 mPa·s, en particulier dans la plage allant de 50 à 15 000 mPa·s, de préférence dans la plage allant de 100 à 10 000 mPa·s, de manière davantage préférée dans la plage allant de 500 à 8 000 mPa·s, de manière particulièrement préférée dans la plage allant de 1 000 à 7 000 mPa·s, de manière encore davantage préférée dans la plage allant de 2 000 à 6 000 mPa·s ; et/ou
la composition ayant une valeur de pH physiologiquement acceptable située dans la plage allant de 6 à 7 ; et/ou
la composition ayant une conductivité électrique physiologiquement acceptable située en particulier dans la plage allant de 1 à 20 mS/cm, de préférence dans la plage allant de 2 à 15 mS/cm, de manière davantage préférée dans la plage allant de 3 à 13 mS/cm, de manière particulièrement préférée dans la plage allant de 5 à 10 mS/cm.

8. Composition destinée à être utilisée selon l'une des revendications précédentes,
la composition contenant au moins un acide et/ou une base, en particulier pour former un système tampon et/ou la composition contenant au moins une base, la base étant choisie en particulier dans le groupe constitué par des aminés, des carboxylates, des hydroxydes de métaux alcalins et/ou alcalino-terreux, ainsi que des combinaisons de ceux-ci, en particulier des hydroxydes de métaux alcalins et/ou alcalino-terreux, de préférence de l'hydroxyde de sodium ; et/ou
la composition ne contenant ni agent conservateur ni substance conservatrice et/ou la composition ne contenant au moins pratiquement pas d'agents conservateurs et/ou de substances conservatrices.

9. Composition destinée à être utilisée selon l'une des revendications précédentes,
la composition contenant au moins un autre agent actif et/ou ingrédient choisi en particulier dans le groupe constitué par des agents protecteurs pour la peau, des antiseptiques, des anesthésiques locaux, des vitamines, des oligo-éléments, des minéraux, des micro-nutriments et des combinaisons de ceux-ci ; et/ou
la composition contenant au moins un additif et/ou un auxiliaire pharmaceutique usuel, choisi(s) en particulier dans le groupe constitué par des auxiliaires de préparation, des stabilisateurs, des émulsifiants, des antioxydants, des conservateurs, des agents humectants, des ajusteurs de pH, des substances tampons de pH, des épaississants, des antiseptiques, des substances colorantes, tampons, odorantes, parfumantes, diluantes, liantes, de réticulation et/ou de conservation et des combinaisons de ceux-ci.

10. Composition destinée à être utilisée selon l'une des revendications précédentes, **caractérisée en ce que** la composition est contenue dans un récipient, en particulier un dispositif d'application, de préférence sous la forme préférentielle d'une seringue stérile.

11. Composition destinée à être utilisée selon l'une des revendications précédentes, **caractérisée en ce qu'**un cathéter, en particulier un cathéter urinaire ou un cathéter pour fistule, est enduit avec la composition, la composition enduisant en particulier une section du cathéter pouvant être introduite et/ou insérée dans une lumière corporelle, en particulier dans l'urètre ou dans une fistule artificielle destinée à une dérivation urinaire.

12. Composition destinée à être utilisée selon l'une des revendications précédentes, **caractérisée en ce que** la composition est présente dans un kit, en particulier un système de pose de cathéters, le kit consistant en des composants séparés spatialement comprenant d'une part un cathéter, en particulier un cathéter urinaire ou pour fistule, et d'autre part une composition destinée à être utilisée selon l'une des revendications précédentes, en particulier dans lequel la composition est contenue dans un récipient.
